# EUROPEAN PATENT SPECIFICATION

(11) **EP 4 324 512 B1**
(45) Date of publication and mention of the grant of the patent: **04.02.2026**
(21) Application number: 22787356.9
(22) Date of filing: 25.03.2022
(51) Int. Cl.: A61N 1/36, A61N 1/378

(54) **IMPLANTABLE NEUROSTIMULATOR**
IMPLANTIERBARER NEUROSTIMULATOR
NEUROSTIMULATEUR IMPLANTABLE

(30) Priority: 16.04.2021 CN 202110412272
(43) Date of publication of application: 21.02.2024
(73) Proprietor: BEIJING LEADING INNOVATION MEDICAL VALLEY CO., LTD, Beijing 100162 (CN)
(72) Inventor: XU, Tianrui, Beijing 100162 (CN)
(74) Representative: EP&C
(86) International application number: PCT/CN2022/082968
(87) International publication number: WO 2022/218127

(56) References cited:
- EP-A1- 3 747 507
- CN-A- 101 244 312
- CN-A- 103 845 793
- CN-A- 105 311 750
- CN-A- 111 228 646
- CN-A- 112 972 895
- CN-U- 216 295 009
- US-A1- 2013 238 048
- US-A1- 2018 236 241
- US-A1- 2019 358 460

## Description

### Technical Field

The present disclosure relates to an implantable neurostimulator, which forms a neural stimulation system with an extracorporeal energy controller through radio frequency communication. The present disclosure particularly relates to an implantable neurostimulator with a main control chip.

### Background Art

A neural stimulation system comprising an implantable neurostimulator has been widely used in the medical field. In this system, the implantable neurostimulator is implanted into the patient's body to achieve treatment of the affected area.

A traditional implantable neurostimulator requires a built-in battery for power supply. After the battery runs out of charge, it is necessary to take the neurostimulator out off the patient's body in order to reinstall the battery. In addition, when doctors need to change a treatment regimen, it is also necessary to take the neurostimulator out off the patient's body in order to reconfigure the treatment regimen. The treatment regimen includes, for example, pulse width, frequency, and the like of a stimulation pulse. This is undoubtedly painful for a patient for a long treatment cycle. To address this pain, a neural stimulation system with controlled radio-frequency has emerged, and such neurostimulator system has been disclosed in US patent application US2013238048A1 and Chinese invention patents CN104080509B and CN107789730B, wherein the implantable neurostimulator conducts radio frequency communication and energy transmission with an extracorporeal energy controller which provides an electrical stimulation pulse in real-time to drive a stimulation electrode of the implantable neurostimulator, thereby applying a stimulation signal to the patient's treatment site; and provides radio frequency energy to the implantable neurostimulator so as to maintain operation of the implantable neurostimulator.

Compared to previous implantable neural stimulation systems, the radio-frequency based neurostimulator can obtain almost endless power supply, so there is no need to worry about battery depletion. Moreover, this radio-frequency based implantable neurostimulator can adjust the electrical stimulation pulse at any time by the extracorporeal energy controller according to the treatment regimen. Therefore, there is no need to worry about repeated implantation issues caused by battery depletion and changes in treatment regimen.

However, there are still many shortcomings in the radio-frequency based neural stimulation system of the prior art.

Due to the fact that the extracorporeal energy controller needs to provide electrical energy and input signals (such as various stimulation pulse sequences) simultaneously to the implantable neurostimulator, and real-time monitoring of the working status of the implantable neurostimulator is required, it may not be possible to achieve real-time operation of the implantable neurostimulator, which also has adverse effects on the treatment process. To address this issue, CN107789730B adopts a dual frequency operating mode, which increases the complexity and manufacturing cost of the product and may lead to an increase in size of the implantable neurostimulator. And this increase in size is obviously not conducive to the implantation of neurostimulators.

In addition, since an electrical stimulation pulse of the implantable neurostimulator is provided in real-time by the extracorporeal energy controller, it is necessary to ensure reliable communication between the neurostimulator implanted in the patient's body and the extracorporeal energy controller. And the reliability of this communication will be influenced by many factors. For example, when the extracorporeal energy controller is away from the patient due to some factors, or when the extracorporeal energy controller is accidentally impacted or damaged, even for a very short period of time, it will have adverse effects on the treatment process of the implantable neurostimulator.

The information disclosed in the background part of the present invention is only intended to increase an understanding of the overall background of the present invention, and should not be regarded as acknowledging or implying in any form that the information constitutes the prior art that is already well-known to those skilled in the art.

### Summary of the Invention

A purpose of the present disclosure is to provide an implantable neurostimulator, which forms a neural stimulation system with an extracorporeal energy controller through radio frequency communication. The neural stimulation system may also comprise upper computer software for easy operation and setting, with its characteristics being that the operation control of the implantable neurostimulator is not completed by the extracorporeal energy controller, but by a main control chip built in the implantable neurostimulator. This overcomes issues of treatment safety and product complexity caused by the need for real-time communication in implantable nerve stimulator systems of the prior art.

Specifically, the present disclosure provides an implantable neurostimulator that communicates with and receives electrical energy from an extracorporeal energy controller through radio frequency, comprising: a main control chip comprising a main CPU, a main memory, and a digital to analog conversion current source circuit (DAC); a stimulator antenna and its impedance matching circuit, which are radio-frequency coupled with the extracorporeal energy controller to receive input signals containing electrical energy and control information from the extracorporeal energy controller, and are capable of sending data to the extracorporeal energy controller; a rectification energy storage circuit connected to the impedance matching circuit and the main control chip respectively, so as to extract electrical energy from the received input signal and store electrical energy, and supply power to the main control chip; a modulation/demodulation circuit connected to the impedance matching circuit and the main control chip to extract control information from the received input signal and transmit the control information to the main control chip, and configured to modulate data from the main control chip, which is then transmit to the impedance matching circuit, and sent to the extracorporeal energy controller through the stimulator antenna; an electrode interface connected to the main control chip and configured to receive polarity assignment information from the main control chip, and receives stimulation pulse sequences from the digital to analog conversion current source circuit; one or more stimulation electrodes connected to the electrode interface which assigns the stimulation pulse sequences to each corresponding stimulation electrode based on the polarity assignment information; wherein the main memory stores a control program and the received control information, the main control CPU runs the control program to control the digital to analog conversion current source circuit to generate the stimulation pulse sequences based on the control information, and the control information comprises a combination of clinical stimulation parameters which is a combination of parameters consisting of polarity assignment information parameters, pulse width parameters, pulse amplitude parameters and pulse frequency parameters.

In the above implantable neurostimulator, preferably the main memory is a non-volatile memory.

In the above implantable neurostimulator, preferably the combination of clinical stimulation parameters comprise multiple groups of the clinical stimulation parameters, each group of which havs its own code, and the control information further comprises codes of clinical stimulation parameters which correspond one-by-one with the codes of multiple groups of the combination of clinical stimulation parameters stored in the main memory.

In the above implantable neurostimulator, preferably the control information further comprises an adding/subtracting instruction, in response to which the main control chip adjusts pulse intensity of the stimulation pulse sequence in a step-by-step manner.

In the above implantable neurostimulator, preferably the control information further comprises a data read instruction, in response to which the main control CPU sends corresponding data stored in the main memory to the extracorporeal energy controller.

In the above implantable neurostimulator, preferably the parameters in the combination of clinical stimulation parameters also comprise a charge balance time, the length of which is sufficient to ensure full release of charges between adjacent electrical stimulation pulses, thereby achieving passive charge balance.

In the above implantable neurostimulator, preferably a charge balance circuit is also connected between the electrode interface and the digital to analog conversion current source circuit of the main control chip, which is capable of applying a reverse pulse to the electrode interface between adjacent electrical stimulation pulses, thereby achieving active charge balance.

In the above implantable neurostimulator, preferably it further comprises an operation data memory that is electrically connected to the main control chip for storing various operation data generated during operation of the implantable neurostimulator, and the control information further comprises a data read instruction, in response to which the main control CPU sends data stored in the operation data memory to the extracorporeal energy controller. Further preferably, the operation data memory is a non-volatile memory.

In the above implantable neurostimulator, preferably it further comprises a post measurement feedback circuit that is respectively connected to the electrode interface and the main control chip to measure real-time stimulation parameters on the stimulation electrode and transmit the real-time stimulation parameters to the main control chip, and the main control chip stores the real-time stimulation parameters into the operation data memory.

In the above implantable neurostimulator, preferably the main control chip compares the real-time stimulation parameters with the clinical stimulation parameters, and corrects the stimulation signals applied to each stimulation electrode based on the comparing result.

In the above implantable neurostimulator, preferably it further comprises a front measurement feedback circuit that is provided between the rectification energy storage circuit and the main control chip to measure amount of real-time electrical energy storage in the rectification energy storage circuit at any time, which is transmit to the main control chip and stored in the operation data memory by the main control chip.

In the above implantable neurostimulator, preferably the main control chip evaluates whether it is necessary to adjust the radio-frequency input electrical energy based on the amount of real-time electrical energy storage, when the amount of real-time electrical energy storage is lower than the set value, the main control chip sends a power adjustment instruction to an antenna of the external energy controller through the stimulator antenna and its impedance matching circuit, thereby adjusting the transmission power of the extracorporeal energy controller.

In the above implantable neurostimulator, preferably the main control chip controls the implantable neurostimulator and periodically sends various operation data stored in the operation data memory to the extracorporeal energy controller.

The implantable neurostimulator of the present invention can achieve the following beneficial technical effects. Due to the implementation of electrical pulse stimulation based on the combination of treatment parameters stored in its own main memory, radio-frequency electrical energy needs to be provided only by the extracorporeal energy controller, without the need to obtain real-time stimulation signals containing stimulation electrical pulses from the extracorporeal energy controller. Therefore, the operation reliability of the implantable neurostimulator is improved, and there is no need to worry about treatment failure caused by sudden communication interruption or poor communication.

Due to its own energy storage circuit, it can ensure a short period of electrical energy supply in the event of sudden communication interruption or poor communication, without interrupting treatment.

Due to the fact that the memory of the implantable neurostimulator can store various operating parameters and send these data to the extracorporeal energy controller during treatment intervals or when communication is not busy, it can further ensure smooth communication when communication is needed, thereby improving performance of the device.

The methods and devices of the present invention have other characteristics and advantages that will be apparent from the accompanying drawings and subsequent specific embodiments incorporated herein, or will be described in detail in the accompanying drawings and subsequent specific embodiments, which together serve to explain the specific principles of the present invention.

### Brief Description of Drawings

Figure 1 shows a schematic block diagram of an embodiment of a neural stimulation system comprising an implantable neurostimulator of the present invention.
Figure 2 shows a schematic block diagram of another embodiment of a neural stimulation system comprising an implantable neurostimulator of the present invention.
Figure 3 shows a schematic block diagram of an implantable neurostimulator of the present invention.
Figure 4 shows a schematic block diagram of another implantable neurostimulator of the present invention.

It should be understood that the accompanying drawings do not necessarily have to be drawn to scale, as they illustrate various features of the basic principles of the present invention that have been simplified to some extent. The specific design features of the present invention disclosed herein, including specific dimensions, orientation, positioning, and shape, will be partially determined by application and usage environment for the specific purpose.

In these drawings, in all figures running through the drawings, the reference numerals refer to the same or equivalent parts of the present invention.

### Detailed Description of Embodiments

Now, specific reference will be made to the various embodiments of the present invention, and examples of these embodiments are shown in the accompanying drawings and the following description.

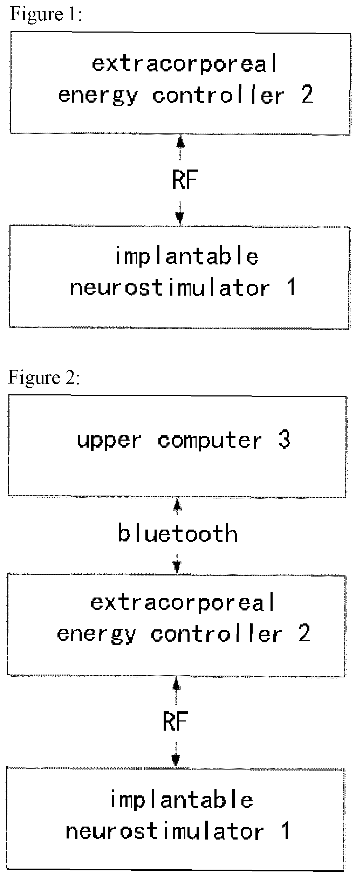

Figure 1 shows a schematic block diagram of an embodiment of a neural stimulation system comprising an implantable neurostimulator of the present invention. As shown in Figure 1, the neural stimulation system comprises two parts: an implantable neurostimulator 1 and an extracorporeal energy controller 2.

Figure 2 shows a schematic block diagram of another embodiment of a neural stimulation system comprising an implantable neurostimulator of the present invention. Compared to the embodiment in Figure 1, the embodiment in Figure 2 is added with an upper computer 3. The upper computer is not necessary. Adding an upper computer helps improve human-computer interaction functions, making it easier for doctors or patients to operate the neural stimulation system and convenient to provide more complex functions for the neural stimulation system.

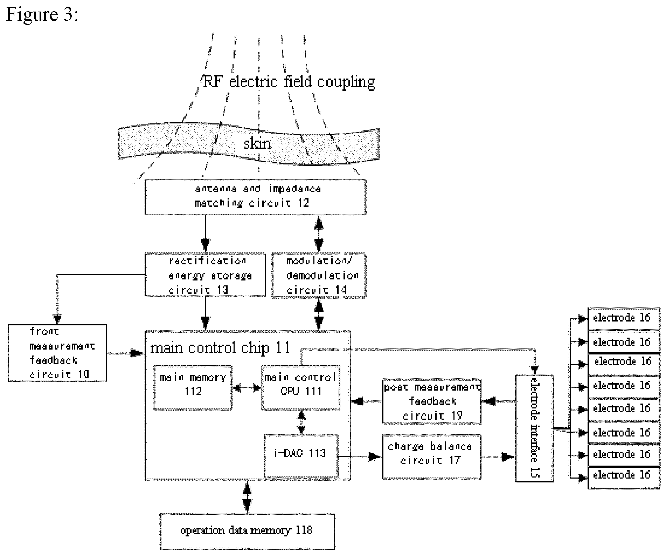

Figure 3 shows a schematic block diagram of an implantable neurostimulator of the present invention.

As shown in Figure 3, an implantable neurostimulator 1 of the present invention that communicates with an extracorporeal energy controller and receives electrical energy from the extracorporeal energy controller through radio frequency, comprises: a main control chip 11 that includes a main CPU 111, a main memory 112, and a digital to analog conversion current source circuit (i-DAC) 113; a stimulator antenna and its impedance matching circuit 12, which are radio-frequency coupled with the extracorporeal energy controller to receive input signals containing electrical energy and control information from the extracorporeal energy controller, and are capable of sending data to the extracorporeal energy controller; a rectification energy storage circuit 13 connected to the impedance matching circuit 12 and the main control chip 11 respectively, so as to extract electrical energy from the received input signal and store electrical energy, and supply power to the main control chip 11; a modulation/demodulation circuit 14 connected to the impedance matching circuit 12 and the main control chip 11 to extract control information from the received input signal and transmit the control information to the main control chip 11, and modulates data sent by the main control chip 11, which is then transmit to the impedance matching circuit, and sent to the extracorporeal energy controller through the stimulator antenna; an electrode interface 15 connected to the main control chip 11 and receives polarity assignment information from the main control chip 11, and receives stimulation pulse sequences from the digital to analog conversion current source circuit 113; one or more stimulation electrodes 16 connected to the electrode interface 15 which assigns the stimulation pulse sequences to each corresponding stimulation electrode 16 based on the polarity assignment information; wherein the main memory 112 is stored with a control program and the received control information, the main control CPU runs the control program to control the digital to analog conversion current source circuit 113 to generate the stimulation pulse sequences based on the control information, and the control information includes a combination of clinical stimulation parameters which is a combination of parameters consisting of polarity assignment information parameters, pulse width parameters, pulse amplitude parameters and pulse frequency parameters.

The main memory 112 is preferably a non-volatile memory, so that data can be stored even after a power outage. In this way, the implantable neurostimulator 1 needs to be configured according to the treatment regimen for each patient only before the start of each treatment stage, which can be applied to the entire treatment stage. Therefore, it avoids the need for doctors to frequently set the implantable neurostimulator 1.

The combination of clinical stimulation parameters comprises multiple groups, each group of which having its own code, and the control information further includes codes of clinical stimulation parameters which correspond one by one with the codes of multiple groups of the combination of clinical stimulation parameters stored in the main memory. In this way, a user (a doctor or a patient) can directly retrieve a corresponding treatment regimen (corresponding to the corresponding combination of the clinical stimulation parameters) by operating the extracorporeal energy controller according to the treatment process, which avoids the hassle of frequently configuring the implantable neurostimulator as the patient's condition improves.

The above control information further includes an adding/subtracting control instruction, in response to which the main control chip 11 adjusts pulse intensity of the stimulation pulse sequence in a step-by-step manner. In this way, the patient can adjust the intensity of stimulation at any time based on its own experience. In the implantable neurostimulator 1, the control information further includes a data read instruction, in response to which the main control CPU sends corresponding data stored in the main memory to the extracorporeal energy controller 2. In this way, the patient or doctor can obtain various combinations of treatment parameters stored in implantable neurostimulator 1, as well as the data generated by operation of the implantable neurostimulator 1.

The implantable neurostimulator uses stimulation pulse sequences to treat patients. When the pulse frequency is high, the charge between adjacent stimulation pulses cannot be fully released, which makes the actual pulse waveform sequence different from the pulse waveform sequence required for the treatment. This will affect the therapeutic effect and also reduce the durability of the implantable neurostimulator itself.

In the implantable neurostimulator 1, the parameters of the combination of clinical stimulation parameters also include a charge balance time, the length of which is sufficient to ensure full release (i.e., discharge) of charges between adjacent electrical stimulation pulses, thereby achieving passive charge balance. This hence overcomes the problem of the existing neurostimulators where the charge between adjacent electrical stimulation pulses cannot be released.

As shown in Figure 3, in the implantable neurostimulator 1, a charge balance circuit 17 is also connected between the electrode interface 15 and the digital to analog conversion current source circuit 113 of the main control chip 11, which is capable of applying a reverse pulse to the electrode interface 15 between adjacent electrical stimulation pulses, thereby achieving active charge balance. Compared to the passive charge balance in natural discharge, the active charge balance can complete the discharge process faster. Obviously, this active charge balance allows for the use of higher stimulation pulse frequencies. In other words, the charge balance circuit 17 is not necessary, depending on the frequency of the stimulation pulse used in implantable neurostimulator 1.

As shown in Figure 3, the implantable neurostimulator 1 also comprises an operation data memory 18 for storing various operation data generated during operation of the implantable neurostimulator. The control information received from the extracorporeal energy controller also includes a data read instruction, in response to which the main control CPU 111 sends the data stored in the operation data memory 18 to the extracorporeal energy controller 2.

It should be noted that the operation data memory 18 is not necessary, and various operation data generated during operation of the implantable neurostimulators can also be stored in a certain partition of the main memory 112, as long as the storage capacity of the main memory is large enough. The operation datamain memory 18 is preferably a non-volatile memory, so that data can be stored even after a power outage. In this way, within the allowable range of storage space, the extracorporeal energy controller can retrieve the operation data of the implantable neurostimulator as needed for a period of time. It also prevents data loss due to sudden interruption of communication.

As shown in Figure 3, the implantable neurostimulator 1 further comprises a post measurement feedback circuit 19 that is respectively connected to the electrode interface 15 and the main control chip 11 to measure real-time stimulation parameters on the stimulation electrode 16, which are transmit to the main control chip 11 and stored in the operation data memory 18 by the main control chip.

The main control chip 11 can compare the real-time stimulation parameters with the clinical stimulation parameters, and corrects the stimulation signals applied to each stimulation electrode based on the comparing result.

It should be noted that the post measurement feedback circuit is not necessary. As a simplified configuration, the implantable neurostimulator can be designed to operate in a simple and reliable mode without the need to measure the working parameters of the stimulation electrode. This helps to reduce costs.

The implantable neurostimulator shown in Figure 3 further comprises a front measurement feedback circuit 10 that is provided between the rectification energy storage circuit 13 and the main control chip 11 to measure amount of real-time electrical energy storage in the rectification energy storage circuit 13 at any time, which is transmit to the main control chip 11 and stored in the operation data memory 18 by the main control chip.

The main control chip 11 evaluates whether it is necessary to adjust the radio-frequency input electrical energy based on the amount of real-time electrical energy storage. When the amount of real-time electrical energy storage is lower than a set value, the main control chip 11 sends a power adjustment instruction to an antenna of the external energy controller 2 through the stimulator antenna and its impedance matching circuit, thereby adjusting the transmission power of the extracorporeal energy controller 2.

As mentioned above, the implantable neurostimulator 1 of the present invention has the main memory and the operation data memory. As a data management measure, the main control chip 11 can actively send data to the extracorporeal energy controller, that is, regularly send various data stored in the main memory and/or operation data memory to the extracorporeal energy controller.

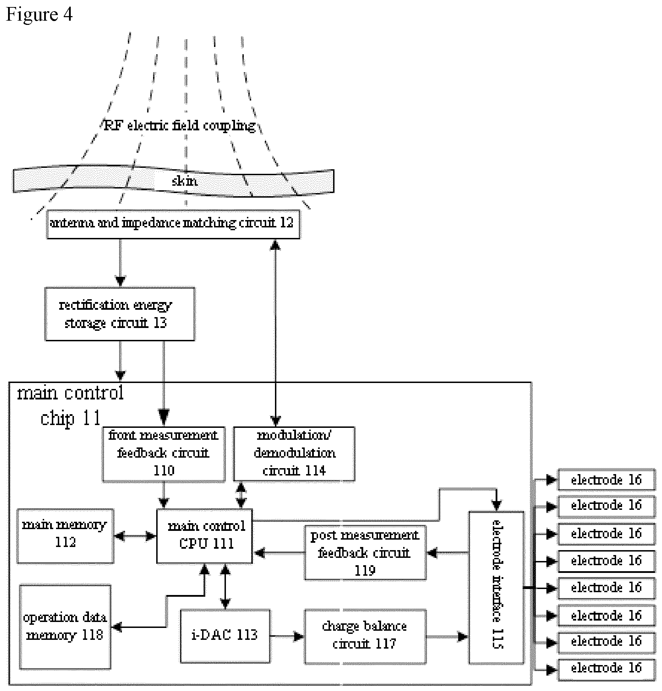

In the schematic block diagram of the implantable neurostimulator shown in Figure 3, the main control chip 11 only includes a main control CPU 111, a main memory 112, and a digital to analog conversion current source circuit (i-DAC) 113, with the circuit part serving as the peripheral circuit. Of course, in order to balance the increase for degree of integration to reduce size and the process cost, some or all of the front measurement feedback circuit, the modulation/demodulation circuit, the electrode interface, the charge balance circuit, the operation data memory, and the post measurement feedback circuit can also be designed in the main control chip 11. For example, in the schematic block diagram of the implantable neurostimulator shown in Figure 4, the main control chip 11 includes the main control CPU 111, the main memory 112, and the digital to analog conversion current source circuit (i-DAC) 113, the front measurement feedback circuit 110, the modulation/demodulation circuit 114, the electrode interface 115, the charge balance circuit 117, the operation data memory 118, and the post measurement feedback circuit 119.

In summary, the implantable neurostimulator 1 of the present invention is configured with parameters by an extracorporeal energy controller and is activated to operate by the extracorporeal energy controller. Once activated, the implantable neurostimulator 1 starts to operate actively with the configured parameters, so as to complete electrode pulse stimulation treatment for the patient.

The implantable neurostimulator 1 of the present invention is self-equipped with a rectification energy storage circuit 13, which stores electrical energy to supply the entire implantable neurostimulator 1 for operation. At the same time, the rectification energy storage circuit 13 receives radio-frequency energy from the extracorporeal energy controller 2 for charging to maintain the continuous operation of the implantable neurostimulator 1. The storage amount of electrical energy in the rectification energy storage circuit 13 can be monitored by the front measurement circuit. When the storage of electrical energy decreases, the implantable neurostimulator 1 will send instructions to extracorporeal energy controller 2, which will increase the transmission power.

As for the implantable neurostimulator 1 of the present invention, due to the implementation of electrical pulse stimulation based on the combination of treatment parameters stored in the main memory, RF electrical energy needs to be provided only by the extracorporeal energy controller, without the need to obtain real-time stimulation signals containing stimulation electrical pulses from the extracorporeal energy controller. Therefore, even if communication is interrupted or blocked due to unexpected events, it will not lead to treatment failure.

Due to its own energy storage circuit, even if the radio frequency power supply is interrupted for a short period of time due to unexpected events, the implantable neurostimulator of the present invention can continue to operate for a period of time until communication returns to normal, thus avoiding interruption during treatment.

Due to the fact that the memory of the implantable neurostimulator can store various operating parameters and send these data to the extracorporeal energy controller during treatment intervals or when communication is not busy, the implantable neurostimulator of the present invention can further ensure smooth communication when communication is needed, thereby improving the performance of device. For example, when a doctor or patient operates the extracorporeal controller to send instructions to the implantable neurostimulator, the implantable neurostimulator will not send data outward so as to ensure smooth communication.

The aforementioned description of the specific exemplary embodiments of the present invention is for the purpose of illustration and exemplification. The purpose of selecting and describing exemplary embodiments is to explain the specific principles and practical applications of the present invention, so that other technical personnel in the art can implement and utilize various exemplary embodiments of the present invention, as well as various choices and changes. The scope of the present invention is intended to be limited by the accompanying claims

## Claims

1. An implantable neurostimulator (1) which communicates with and receives electrical energy from an extracorporeal energy controller (2) through radio frequency, comprising:
a main control chip (11) comprising a main CPU (111), a main memory (112), and a digital to analog conversion current source circuit (113);
a stimulator antenna and its impedance matching circuit (12), which are radio-frequency coupled with the extracorporeal energy controller (2) to receive input signals containing electrical energy and control information from the extracorporeal energy controller (2), and are capable of sending data to the extracorporeal energy controller (2);
a rectification energy storage circuit (13) connected to the impedance matching circuit (12) and the main control chip (11) respectively, so as to extract electrical energy from the received input signal and store electrical energy, and supply power to the main control chip (11);
a modulation/demodulation circuit (14) connected to the impedance matching circuit (12) and the main control chip (11) to extract control information from the received input signal and transmit the control information to the main control chip (11), and configured to modulate the data from the main control chip (11), which is then transmit to the impedance matching circuit (12), and then sent to the extracorporeal energy controller (2) through the stimulator antenna;
an electrode interface (15) connected to the main control chip (11) and configured to receive polarity assignment information from the main control chip (11) and receive stimulation pulse sequences from the digital to analog conversion current source circuit (113);
one or more stimulation electrodes (16) connected to the electrode interface (15) which assigns the stimulation pulse sequences to each corresponding stimulation electrode (16) based on the polarity assignment information;
wherein the main memory (112) is stored with a control program and the received control information, the main control CPU (111) runs the control program to control the digital to analog conversion current source circuit (113) to generate the stimulation pulse sequences based on the control information, and the control information includes combinations of clinical stimulation parameters which is a combination of parameters consisting of polarity assignment information parameters, pulse width parameters, pulse amplitude parameters and pulse frequency parameters;
wherein the control information further includes adding/subtracting instructions, in response to which the main control chip (11) adjusts pulse intensity of the stimulation pulse sequence in a step-by-step manner.

2. The implantable neurostimulator according to claim 1, wherein the main memory (11) is a non-volatile memory.

3. The implantable neurostimulator according to claim 1, wherein the combinations of clinical stimulation parameters comprises multiple groups, each group of which having its own code, and the control information further includes codes of clinical stimulation parameters which correspond one by one with the codes of multiple groups of the combinations of clinical stimulation parameters stored in the main memory (112).

4. The implantable neurostimulator according to claim 1, wherein the control information further includes a data read instruction, in response to which the main control CPU (111) sends corresponding data stored in the main memory (112) to the extracorporeal energy controller (2).

5. The implantable neurostimulator according to claim 1, wherein the parameters of the combination of clinical stimulation parameters futher include a charge balance time, the length of which is sufficient to ensure full release of charges between adjacent electrical stimulation pulses, thereby achieving passive charge balance.

6. The implantable neurostimulator according to claim 1, wherein a charge balance circuit (17) is further connected between the electrode interface (15) and the digital to analog conversion current source circuit (113) of the main control chip (11), which is capable of applying a reverse pulse to the electrode interface (15) between adjacent electrical stimulation pulses, thereby achieving active charge balance.

7. The implantable neurostimulator according to claim 1, further comprising an operation data memory (18) which is electrically connected to the main control chip (11) for storing various operation data generated during operation of the implantable neurostimulator (1), and the control information further includes a data read instruction, in response to which the main control CPU (111) sends data stored in the operation data memory to the extracorporeal energy controller (2).

8. The implantable neurostimulator according to claim 7, wherein the operation data memory (18) is a non-volatile memory.

9. The implantable neurostimulator according to claim 7, further comprising a post measurement feedback circuit (19) which is respectively connected to the electrode interface (15) and the main control chip (11) to measure real-time stimulation parameters on the stimulation electrode (16), which are transmit to the main control chip (11) and stored in the operation data memory (18) by the main control chip (11).

10. The implantable neurostimulator according to claim 9, wherein the main control chip (11) compares the real-time stimulation parameters with the clinical stimulation parameters, and corrects the stimulation signals applied to each stimulation electrode (16) based on the comparing result.

11. The implantable neurostimulator according to claim 7, further comprising a front measurement feedback circuit (10) which is provided between the rectification energy storage circuit (13) and the main control chip (11) to measure amount of real-time electrical energy storage in the rectification energy storage circuit (13) at any time, which is transmit to the main control chip (11) and stored in the operation data memory (18) by the main control chip (11).

12. The implantable neurostimulator according to claim 11, wherein the main control chip (11) evaluates whether it is necessary to adjust the radio-frequency input electrical energy based on the amount of real-time electrical energy storage, when the amount of real-time electrical energy storage is lower than the set value, the main control chip (11) sends a power adjustment instruction to an antenna of the external energy controller (2) through the stimulator antenna and its impedance matching circuit (12), thereby adjusting the transmission power of the extracorporeal energy controller (2).

13. The implantable neurostimulator according to claim 7, wherein the main control chip (11) controls the implantable neurostimulator (1) and periodically sends various operation data stored in the operation data memory (18) to the extracorporeal energy controller (2).

## Patentansprüche

1. Implantierbarer Neurostimulator (1), der über Radiofrequenz mit einer extrakorporalen Energiesteuerung (2) kommuniziert und elektrische Energie davon empfängt, umfassend:
einen Hauptsteuerchip (11), der eine Haupt-CPU (111), einen Hauptspeicher (112) und eine Digital-Analog-Wandler-Stromquellenschaltung (113) umfasst;
eine Stimulatorantenne und ihre Impedanzanpassungsschaltung (12), die mit der extrakorporalen Energiesteuerung (2) Radiofrequenz-gekoppelt sind, um Eingangssignale, die elektrische Energie und Steuerinformationen enthalten, von der extrakorporalen Energiesteuerung (2) zu empfangen, und fähig sind, Daten an die extrakorporale Energiesteuerung (2) zu senden;
eine Gleichrichter-Energiespeicherschaltung (13), die mit der Impedanzanpassungsschaltung (12) und dem Hauptsteuerchip (11) verbunden ist, um elektrische Energie aus dem empfangenen Eingangssignal zu extrahieren und elektrische Energie zu speichern und dem Hauptsteuerchip (11) Leistung zuzuführen;
eine Modulations-/Demodulationsschaltung (14), die mit der Impedanzanpassungsschaltung (12) und dem Hauptsteuerchip (11) verbunden ist, um Steuerinformationen aus dem empfangenen Eingangssignal zu extrahieren und die Steuerinformationen an den Hauptsteuerchip (11) zu übertragen, und dafür gestaltet ist, die Daten von dem Hauptsteuerchip (11) zu modulieren, die dann an die Impedanzanpassungsschaltung (12) übertragen werden und dann durch die Stimulatorantenne zu der extrakorporalen Energiesteuerung (2) gesendet werden;
eine Elektrodenschnittstelle (15), die mit dem Hauptsteuerchip (11) verbunden ist und dafür gestaltet ist, Polaritätszuweisungsinformationen von dem Hauptsteuerchip (11) zu empfangen und Stimulationsimpulssequenzen von der Digital-Analog-Wandler-Stromquellenschaltung (113) zu empfangen;
eine oder mehrere Stimulationselektroden (16), die mit der Elektrodenschnittstelle (15) verbunden sind, die die Stimulationsimpulssequenzen jeder entsprechenden Stimulationselektrode (16) auf der Grundlage den Polaritätszuweisungsinformationen zuordnet;
wobei in dem Hauptspeicher (112) ein Steuerprogramm und die empfangenen Steuerinformationen gespeichert sind, die Hauptsteuer-CPU (111) das Steuerprogramm ausführt, um die Digital-Analog-Wandler-Stromquellenschaltung (113) zu steuern, um die Stimulationsimpulssequenzen auf der Grundlage der Steuerinformationen zu erzeugen, und die Steuerinformationen Kombinationen von klinischen Stimulationsparametern enthalten, die eine Kombination von Parametern sind, die aus Polaritätszuweisungsinformations-Parametern, Impulsbreitenparametern, Impulsamplitudenparametern und Impulsfrequenzparametern bestehen;
wobei die Steuerinformationen ferner Addieren/Subtrahieren von Anweisungen enthalten, wobei als Reaktion darauf der Hauptsteuerchip (11) die Impulsintensität der Stimulationsimpulssequenz auf eine Schritt-für-Schritt-Weise anpasst.

2. Implantierbarer Neurostimulator nach Anspruch 1, wobei der Hauptspeicher (11) ein nichtflüchtiger Speicher ist.

3. Implantierbarer Neurostimulator nach Anspruch 1, wobei die Kombinationen von klinischen Stimulationsparametern mehrere Gruppen umfassen, von denen jede Gruppe einen eigenen Code aufweist, und die Steuerinformationen ferner Codes von klinischen Stimulationsparametern enthalten, die eins-zu-eins den Codes mehrerer Gruppen der Kombinationen von klinischen Stimulationsparametern entsprechen, die in dem Hauptspeicher gespeichert sind (112).

4. Implantierbarer Neurostimulator nach Anspruch 1, wobei die Steuerinformationen ferner eine Datenleseanweisung enthalten, wobei als Reaktion darauf die Hauptsteuer-CPU (111) entsprechende Daten, die in dem Hauptspeicher (112) gespeichert sind, an die extrakorporale Energiesteuerung (2) sendet.

5. Implantierbarer Neurostimulator nach Anspruch 1, wobei die Parameter der Kombination von klinischen Stimulationsparametern ferner eine Ladungsausgleichszeit enthalten, deren Länge ausreicht, vollständige Freisetzung von Ladungen zwischen benachbarten elektrischen Stimulationsimpulsen sicherzustellen, um passiven Ladungsausgleich zu erzielen.

6. Implantierbarer Neurostimulator nach Anspruch 1, wobei ferner zwischen die Elektrodenschnittstelle (15) und die Digital-Analog-Wandler-Stromquellenschaltung (113) des Hauptsteuerchips (11) eine Ladungsausgleichsschaltung (17) geschaltet ist, die fähig ist, einen Umkehrimpuls an die Elektrodenschnittstelle (15) zwischen benachbarten elektrischen Stimulationsimpulsen anzulegen, um aktiven Ladungsausgleich zu erzielen.

7. Implantierbarer Neurostimulator nach Anspruch 1, ferner umfassend einen Betriebsdatenspeicher (18), der elektrisch mit dem Hauptsteuerchip (11) verbunden ist, um verschiedene Betriebsdaten zu speichern, die während des Betriebs des implantierbaren Neurostimulators (1) erzeugt werden, und die Steuerinformationen ferner eine Datenleseanweisung enthalten, wobei als Reaktion darauf die Hauptsteuerungs-CPU (111) Daten, die in dem Betriebsdatenspeicher gespeichert sind, an die extrakorporale Energiesteuerung (2) sendet.

8. Implantierbarer Neurostimulator nach Anspruch 7, wobei der Betriebsdatenspeicher (18) ein nichtflüchtiger Speicher ist.

9. Implantierbarer Neurostimulator nach Anspruch 7, ferner umfassend eine Nachmessungs-Rückkopplungsschaltung (19), die mit der Elektrodenschnittstelle (15) und dem Hauptsteuerchip (11) verbunden ist, um Echtzeit-Stimulationsparameter an der Stimulationselektrode (16) zu messen, die an den Hauptsteuerchip (11) übertragen und durch den Hauptsteuerchip (11) in dem Betriebsdatenspeicher (18) gespeichert werden.

10. Implantierbarer Neurostimulator nach Anspruch 9, wobei der Hauptsteuerchip (11) die Echtzeit-Stimulationsparameter mit den klinischen Stimulationsparametern vergleicht und die Stimulationssignale, die an jede Stimulationselektrode (16) angelegt werden, auf der Grundlage des Vergleichsergebnisses korrigiert.

11. Implantierbarer Neurostimulator nach Anspruch 7, ferner umfassend eine vordere Messrückkopplungsschaltung (10), die zwischen der Gleichrichter-Energiespeicherschaltung (13) und dem Hauptsteuerchip (11) bereitgestellt ist, um die Menge an elektrischer Echtzeit-Energiespeicherung in der Gleichrichter-Energiespeicherschaltung (13) zu jedem Zeitpunkt zu messen, die an den Hauptsteuerchip (11) übertragen und durch den Hauptsteuerchip (11) in dem Betriebsdatenspeicher (18) gespeichert wird.

12. Implantierbarer Neurostimulator nach Anspruch 11, wobei der Hauptsteuerchip (11) bewertet, ob es erforderlich ist, die elektrische Radiofrequenz-Eingangsenergie auf der Grundlage der Menge an elektrischer Echtzeit-Energiespeicherung anzupassen, wenn die Menge an elektrischer Echtzeit-Energiespeicherung niedriger als der Sollwert ist, wobei der Hauptsteuerchip (11) durch die Stimulatorantenne und ihre Impedanzanpassungsschaltung (12) eine Leistungsanpassungsanweisung an eine Antenne der externen Energiesteuerung (2) sendet, um die Sendeleistung der extrakorporalen Energiesteuerung (2) einzustellen.

13. Implantierbarer Neurostimulator nach Anspruch 7, wobei der Hauptsteuerchip (11) den implantierbaren Neurostimulator (1) steuert und periodisch verschiedene in dem Betriebsdatenspeicher (18) gespeicherte Betriebsdaten an die extrakorporale Energiesteuerung (2) sendet.

## Revendications

1. Neurostimulateur implantable (1) qui communique avec un dispositif de commande d'énergie extracorporelle (2) et reçoit de l'énergie électrique de celui-ci par radiofréquence, comprenant:
une puce de commande principale (11) comprenant une unité centrale principale (111), une mémoire principale (112) et un circuit source de courant de conversion numérique-analogique (113);
une antenne de stimulateur et son circuit d'adaptation d'impédance (12), qui sont couplés par radiofréquence au dispositif de commande d'énergie extracorporelle (2) pour recevoir des signaux d'entrée contenant de l'énergie électrique et des informations de commande provenant du dispositif de commande d'énergie extracorporelle (2), et sont susceptibles d'envoyer des données au dispositif de commande d'énergie extracorporelle (2);
un circuit de stockage d'énergie de redressement (13) connecté au circuit d'adaptation d'impédance (12) et à la puce de commande principale (11) respectivement, de manière à extraire de l'énergie électrique du signal d'entrée reçu et à stocker l'énergie électrique, et à fournir une puissance à la puce de commande principale (11);
un circuit de modulation/démodulation (14) connecté au circuit d'adaptation d'impédance (12) et à la puce de commande principale (11) pour extraire des informations de commande du signal d'entrée reçu et transmettre les informations de commande à la puce de commande principale (11), et configuré pour moduler les données de la puce de commande principale (11), qui sont ensuite transmises au circuit d'adaptation d'impédance (12), puis envoyées au dispositif de commande d'énergie extracorporelle (2) par le biais de l'antenne de stimulateur;
une interface d'électrode (15) connectée à la puce de commande principale (11) et configurée pour recevoir des informations d'attribution de polarité provenant de la puce de commande principale (11) et recevoir des séquences d'impulsions de stimulation provenant du circuit source de courant de conversion numérique-analogique (113);
une ou plusieurs électrodes de stimulation (16) connectées à l'interface d'électrodes (15) qui attribue les séquences d'impulsions de stimulation à chaque électrode de stimulation (16) correspondante sur la base des informations d'attribution de polarité;
dans lequel la mémoire principale (112) stocke un programme de commande et les informations de commande reçues, l'unité centrale de commande principale (111) exécute le programme de commande pour commander le circuit source de courant de conversion numérique-analogique (113) pour générer les séquences d'impulsions de stimulation sur la base des informations de commande, et les informations de commande comportent des combinaisons de paramètres de stimulation cliniques qui sont une combinaison de paramètres consistant en paramètres d'informations d'attribution de polarité, paramètres de largeur d'impulsion, paramètres d'amplitude d'impulsion et paramètres de fréquence d'impulsion;
dans lequel les informations de commande comportent en outre des instructions d'addition/soustraction, en réponse auxquelles la puce de commande principale (11) règle l'intensité d'impulsion de la séquence d'impulsions de stimulation pas à pas.

2. Neurostimulateur implantable selon la revendication 1, dans lequel la mémoire principale (11) est une mémoire non volatile.

3. Neurostimulateur implantable selon la revendication 1, dans lequel les combinaisons de paramètres de stimulation clinique comprennent des groupes multiples, dont chaque groupe a son propre code, et les informations de commande comportent en outre des codes de paramètres de stimulation clinique qui correspondent un par un aux codes de groupes multiples des combinaisons de paramètres de stimulation clinique stockés dans la mémoire principale (112).

4. Neurostimulateur implantable selon la revendication 1, dans lequel les informations de commande comportent en outre une instruction de lecture de données, en réponse à laquelle l'unité centrale de commande principale (111) envoie des données correspondantes stockées dans la mémoire principale (112) au dispositif de commande d'énergie extracorporelle (2).

5. Neurostimulateur implantable selon la revendication 1, dans lequel les paramètres de la combinaison de paramètres de stimulation clinique comportent en outre un temps d'équilibre de charge, dont la longueur est suffisante pour assurer une libération complète des charges entre des impulsions de stimulation électrique adjacentes, réalisant de ce fait un équilibre de charge passif.

6. Neurostimulateur implantable selon la revendication 1, dans lequel un circuit d'équilibe de charge (17) est en outre connecté entre l'interface d'électrode (15) et le circuit source de courant de conversion numérique-analogique (113) de la puce de commande principale (11), qui est susceptible d'appliquer une impulsion inverse à l'interface d'électrode (15) entre des impulsions de stimulation électrique adjacentes, réalisant de ce fait un équilibre de charge actif.

7. Neurostimulateur implantable selon la revendication 1, comprenant en outre une mémoire de données de fonctionnement (18) qui est connectée électriquement à la puce de commande principale (11) pour stocker diverses données de fonctionnement générées pendant le fonctionnement du neurostimulateur implantable (1), et les informations de commande comportent en outre une instruction de lecture de données, en réponse à laquelle l'unité centrale de commande principale (111) envoie des données stockées dans la mémoire de données de fonctionnement au dispositif de commande d'énergie extracorporelle (2).

8. Neurostimulateur implantable selon la revendication 7, dans lequel la mémoire de données de fonctionnement (18) est une mémoire non volatile.

9. Neurostimulateur implantable selon la revendication 7, comprenant en outre un circuit de rétroaction de post-mesure (19) qui est respectivement connecté à l'interface d'électrode (15) et à la puce de commande principale (11) pour mesurer des paramètres de stimulation en temps réel sur l'électrode de stimulation (16), qui sont transmis à la puce de commande principale (11) et stockés dans la mémoire de données de fonctionnement (18) par la puce de commande principale (11).

10. Neurostimulateur implantable selon la revendication 9, dans lequel la puce de commande principale (11) compare les paramètres de stimulation en temps réel aux paramètres de stimulation clinique, et corrige les signaux de stimulation appliqués à chaque électrode de stimulation (16) sur la base du résultat de comparaison.

11. Neurostimulateur implantable selon la revendication 7, comprenant en outre un circuit de rétroaction de mesure frontale (10) qui est prévu entre le circuit de stockage d'énergie de redressement (13) et la puce de commande principale (11) pour mesurer la quantité de stockage d'énergie électrique en temps réel dans le circuit de stockage d'énergie de redressement (13) à tout moment, qui est transmise à la puce de commande principale (11) et stockée dans la mémoire de données de fonctionnement (18) par la puce de commande principale (11).

12. Neurostimulateur implantable selon la revendication 11, dans lequel la puce de commande principale (11) évalue s'il est nécessaire d'ajuster l'énergie électrique d'entrée radiofréquence sur la base de la quantité de stockage d'énergie électrique en temps réel, lorsque la quantité de stockage d'énergie électrique en temps réel est inférieure à la valeur fixée, la puce de commande principale (11) envoie une instruction d'ajustement de puissance à une antenne du dispositif de commande d'énergie externe (2) par le biais de l'antenne de stimulateur et de son circuit d'adaptation d'impédance (12), ajustant de ce fait la puissance de transmission du dispositif de commande d'énergie extracorporelle (2).

13. Neurostimulateur implantable selon la revendication 7, dans lequel la puce de commande principale (11) commande le neurostimulateur implantable (1) et envoie périodiquement diverses données de fonctionnement stockées dans la mémoire de données de fonctionnement (18) au dispositif de commande d'énergie extracorporelle (2).
